# EUROPÄISCHE PATENTSCHRIFT

(11) **EP 1 123 980 B1**
(45) Veröffentlichungstag und Bekanntmachung des Hinweises auf die Patenterteilung: **14.10.2009**
(21) Anmeldenummer: 01103113.5
(22) Anmeldetag: 09.02.2001
(51) Int. Cl.: C12Q 1/68, B01L 7/00, B01L 3/00, B01J 19/00

(54) **System zur einfachen Nukleinsäureanalytik**
System for simple nucleic acid analysis
Système d'analyse simple d'acides nucléiques

(30) Priorität: 11.02.2000 DE 10006214
(43) Veröffentlichungstag der Anmeldung: 16.08.2001
(73) Patentinhaber: Roche Diagnostics GmbH, 68305 Mannheim (DE); F. Hoffmann-La Roche AG, 4070 Basel (CH)
(72) Erfinder: Fritz, Michael, 68647 Biblis (DE); Harttig, Herbert, Dr., 67122 Altrip (DE); Steinbiss, Joachim, Dr., 64653 Lorsch (DE); Gerstle, Volker, 64653 Lorsch (DE); Schwab, Jürgen, 68775 Ketsch (DE); Rauscher, Andreas, Dr., 67071 Ludwigshafen (DE)
(74) Vertreter: Dey, Michael

(56) Entgegenhaltungen:
- DE-B- 2 343 987
- US-A- 5 415 839
- US-A- 5 635 358
- US-A- 5 714 380
- US-A- 5 849 208
- US-A- 5 955 351

### SEQUENZPROTOKOLL

<110> Roche Diagnostics GmbH (Mannheim)
<120> System zur einfachen Nukleinsäureanalytik
<130> 20913PEP_DR
<140> 01103113.5
   <141> 2001-02-09
<150> DE 100 06 214.8
   <151> 2000-02-11
<160> 3
<170> PatentIn Ver. 2.1
<210> 1
   <211> 25
   <212> DNA
   <213> Künstliche Sequenz
<220>
   <223> Beschreibung der künstlichen Sequenz:
   Oligonukleotid CP24 (Position 195-219 von pCTT1)
<400> 1
   gggattcctg taacaacaag tcagg 25
<210> 2
   <211> 26
   <212> DNA
   <213> Künstliche Sequenz
<220>
   <223> Beschreibung der künstlichen Sequenz:
   Oligonukleotid CP24 (Position 401-376 von pCTT1)
<400> 2
   cctcttcccc agaacaataa gaacac 26
<210> 3
   <211> 21
   <212> DNA
   <213> Künstliche Sequenz
<220>
   <223> Beschreibung der künstlichen Sequenz:
   Nachweissonde aus dem Chlamydia trachomatis
   Plasmid (Position 354-374)
<400> 3
   gtctctcatc gagacaaagt g 21

## Beschreibung

Die vorliegende Erfindung betrifft ein System zur einfachen Nukleinsäureanalytik.

Bei bekannten Systemen zur Nukleinsäurenanalytik wird zunächst eine Bindung von Nukleinsäuren zur Reinigung bzw. Isolierung des gesuchten Analyten durchgeführt. Die Bindung erfolgt üblicherweise in Gefäßen mit relativ großen Volumina von einigen cm³, um die für einen sensitiven Nachweis erfoderliche Menge an Probe, Bindepuffer etc. aufnehmen zu können. Da im diagnostischen Bereich Nachweisgrenzen von 1 bis 10 Analyten pro ml Probe erreicht werden sollen, sind für sensitive diagnostische Verfahren entsprechend große Probemengen und damit große Reaktionsvolumina erforderlich. Kleinere Probevolumina führen zu weniger sensitiven Verfahren und zu der Gefahr, daß in dem ausgewählten Teil der tatsächlich positiven Probe der gesuchte Analyt gerade nicht vorhanden ist, was zu einem falsch negativen diagnostischen Ergebnis führen würde.

Andererseits sollte eine für den Nachweis erforderliche Nukleinsäureamplifikation des isolierten DNA-Analyten jedoch in möglichst kleinen Gefäßen durchgeführt werden, um eine ausreichend hohe Amplifikationsgeschwindigkeit zu erzielen. Dieser Kontrast hat zur Folge, daß die zur Reinigung immobilisierten Nukleinsäuren üblicherweise aus dem Bindungsraum eluiert und in einen Amplifikationsraum überführt werden müssen, der vom Bindungsraum verschieden ist. Vorrichtungen für eine Nukleinsäureanalytik mit Mehrkammersystemen werden beispielweise in EP 0 754 725; WO 95/11454; US 5,645,801; WO 97/02357; US 5,714,380; EP 0 733 714; EP 0 674 009; US 5,725,831; US 5,639,428; WO 97/10056; WO 94/05414; WO 96/ 41864; US 5,589,136; WO 97/00726; EP 0 838 025; WO 97/03348; EP 0 594 260; EP 0 594 259; US 5,288,463; US 5,422,271; US 5,593,838; WO 93/22053; WO 93/22054; WO 93/22055; WO 93/22058 und WO 96/15269 beschrieben. Der bei diesen Vorrichtungen erforderliche Transferschritt der isolierten Nukleinsäuren von einem Gefäß in ein anderes, ist jedoch mit zusätzlichen Maßnahmen und der Gefahr verbunden, bei der Überführung einen Teil oder den gesamten gesuchten Analyten zu verlieren oder die Probe zu kontaminieren.

US 5,955,351 beschreibt eine in sich geschlossene, mehrkammerige Vorrichtung zur Nukleinsäureanalyse, wobei eine Nukleinsäureextraktion, -amplifikation und -detektion in mehreren zueinander beweglichen Reaktionsräumen erfolgt. Die Aufbereitung und die Detektion der zu untersuchenden Nukleinsäuren erfolgt innerhalb der Vorrichtung in voneinander separierten Kammern. Das zu analysierende Probenvolumen ist hierbei auf das Volumen des Bindungsraums beschränkt.

Es besteht deshalb ein Bedarf nach Nukleinsäureanalyseverfahren, mit denen die Nachteile der bekannten Verfahren überwunden werden können und die insbesondere eine hohe Sensitivität aufweisen und mit denen der technische und zeitliche Aufwand für eine Analyse verringert werden kann.

Diese Aufgabe wird erfindungsgemäß gelöst durch ein Verfahren zum Nachweis von Nukleinsäuren in einer Probe umfassend die Schritte:
(a) Reinigen der Nukleinsäuren in einem Bindungsraum, wobei die Nukleinsäuren immobilisiert und Verunreinigungen abgetrennt werden,
(b) Eluieren der immobilisierten Nukleinsäuren,
(c) Amplifizieren der gereinigten Nukleinsäuren in einem Amplifikationsraum, wobei der Amplifikationsraum mindestens einen Teil des Bindungsraums umfasst, und
(d) Detektieren der Amplifikationsprodukte in einem Detektionsraum,
welches dadurch gekennzeichnet ist, daß der Detektionsraum mindestens einen Teil des Amplifikationsraums oder/und einen Teil des Bindungsraums umfasst.

Aufgrund der Durchführung der Amplifikation in mindestens einem Teil des Bindungsraumes, kann eine deutliche Verbesserung im Hinblick auf den technischen und zeitlichen Aufwand bei der Nukleinsäureanalytik erhalten werden. Erfindungsgemäß kann der gleiche Raum oder ein Teil des Raumes sowohl für die Immobilisierung als auch die Amplifikation verwendet werden, wobei die Probe, gegebenenfalls vermischt mit weiteren Reagenzien, zur Immobilisierung mindestens einmal, bevorzugt mehrmals, durch den Bindungsraum hindurchgeleitet wird.

Grundsätzlich umfassen Systeme zur Nukleinsäureanalytik einen oder mehrere der folgenden Schritte: Probenvorbereitung, Amplifikation, Detektion und Auswertung.

Die Probenvorbereitung kann gegebenenfalls vor dem Reinigen der Nukleinsäure in Schritt (a) eine Lyse von nukleinsäurehaltigen Proben umfassen. Eine solche Lyse wird bevorzugt durchgeführt, wenn die zu untersuchende Probe Zellen oder/und Zellbestandteile enthält. Eine Lyse von Zellen kann beispielsweise durch Zugabe von lytischen Reagenzien, insbesondere chaotropen Reagenzien oder lytischen Enzymen bewerkstelligt werden. In einer besonders bevorzugten Ausführungsform wird in einer Doppelkolbenspritze ein chaotropes Reagenz vorgelegt. Die Probe wird in die Spritze aufgezogen und die Spritzenöffnung anschließend verschlossen, beispielsweise durch ein Ventil oder einen Hahn. Durch Hin- und Herbewegen des zweiten Kolbens der Doppelkolbenspritze wird eine intensive Durchmischung von nukleinsäurehaltiger Probe und chaotropem Reagenz erreicht. Falls gewünscht, kann die Lyse durch Erwärmung der Mischung bei erhöhter Temperatur, bevorzugt von 30°C bis 70°C durchgeführt werden. Die Lyse wird insbesondere durch Ausübung von Scherkräften auf die nukleinsäurehaltige Probe bewirkt.

Weiterhin umfaßt die Probenvorbereitung einen Reinigungsschritt, um möglicherweise den späteren Nachweis störende Komponenten der Probe abzutrennen. Die Reinigung der Nukleinsäuren wird erfindungsgemäß in einem Bindungsraum durchgeführt, wobei die Nukleinsäuren immobiliert und Verunreinigungen abgetrennt werden. Die Nukleinsäuren können z.B. durch kovalentes oder adsorptives Binden im Bindungsraum immobilisiert werden. Weiterhin ist auch eine Bindung der Nukleinsäuren über hochaffine Wechselwirkungen, beispielsweise eine sequenzspezifische Bindung über Hybridisierungssonden oder über Bindepartner von hochaffinen Bindungspaaren wie etwa Avidin/Biotin oder über spezifische Antikörper möglich. Bevorzugt erfolgt die Immobilisierung der Nukleinsäuren durch adsorptive Bindung, beispielsweise an eine Glasoberfläche. Die Bindung der Nukleinsäuren an die Oberfläche kann durch Zugabe von geeigneten Bindereagenzien, wie etwa Isopropanol, chaotropen Salzen etc. unterstützt werden.

Die Bindung erfolgt bevorzugt an eine innere Oberfläche des Bindungsraumes, es ist aber auch möglich, die Nukleinsäuren an im Bindungsraum eingebrachte Anordnungen, wie etwa Packungen oder Schüttungen zu binden.

Falls eine Zellen, beispielsweise Mikroorganismen enthaltende Probe analysiert werden soll, kann es vorteilhaft sein, zunächst die Zellen, z.B. Infektionskeime, über spezifische insbesondere oberflächenantigenspezifische Antikörper oder unspezifische adsorptive Bindung an die Oberfläche des Bindungsraums abzufangen bzw. zu isolieren. So können beispielsweise für eine spezifische Bindung von Mikroorganismen, wie beispielsweise Infektionskeimen entsprechende Antikörper im Bindungsraum immobilisiert werden. Es konnte gezeigt werden, daß Zellen wie beispielsweise Chlamydien adsorbtiv an Oberflächen, bevorzugt Oberflächen aus Polystyrol gebunden und auf diese Weise aufgereinigt werden können. Die Nukleinsäuren selbst können dann nach einer Lyse der Zellen, die beispielsweise durch thermische Einwirkung während der Amplifikationsreaktion erfolgen kann, nachgewiesen werden.

Besonders bevorzugt wird als Bindungsraum ein zumindest teilweise kapillarer Raum verwendet. In einer bevorzugten Ausführungsform erfolgt die Immobilisierung der Nukleinsäuren oder Zellen an die innere Oberfläche einer Kapillare, die aus Glas, insbesondere Borsilikat, oder Polystyrol besteht. Der kapillare Raum kann aber auch als Spalt, z.B. zwischen zwei Deckplatten ausgebildet sein. Der Öffnung bzw. der Durchmesser des kapillaren Raums beträgt bevorzugt ≥ 0,05 mm, besonders bevorzugt ≥ 0,5 mm und am meisten bevorzugt ≥ 0,9 mm und bevorzugt ≤ 5 mm, besonders bevorzugt ≤ 2 mm und am meisten bevorzugt ≤ 1,1 mm. Während nur ein Teil des Bindungsraumes, insbesondere ≥ 10%, bevorzugt ≥ 20% des gesamten Bindungsvolumens als kapillarer Raum ausgebildet sein können, ist es bevorzugt, daß praktisch der gesamte Bindungsraum, bevorzugt ≥ 80% und insbesondere ≥ 90% bezogen auf das gesamte Bindungsraumvolumen als kapillarer Raum ausgebildet sind.

Zur Immobilisierung wird eine Kapillare beispielsweise an die oben beschriebene Doppelkolbenspritze angedockt und die nukleinsäurehaltige Probe bzw. das nach einer Lyse erhaltene Gemisch mindestens einmal, bevorzugt mindestens fünfmal, mehr bevorzugt mindestens zehnmal und am meisten bevorzugt mindestens zwanzigmal durch die Kapillare geleitet. Durch das wiederholte Leiten der Probe bzw. der Lysemischung durch den Bindungsraum kann vorteilhafterweise eine Erhöhung der Ausbeute an immobilisierten Nukleinsäuren und damit eine Erhöhung der Sensitivität des gesamten Verfahrens erzielt werden.

Das Volumenverhältnis von Bindungsraum zu Nukleinsäure enthaltender Probe beträgt bevorzugt mindestens 10:1, besonders bevorzugt mindestens 20:1.

Ein großes Verhältnis von Bindungsraum zu Probe ist vorteilhaft, da es ein großes Probevolumen, wie es für eine hohe Sensitivität bei der Bindung erforderlich ist, und gleichzeitig die Durchführung des Verfahrens in einem kleinen Raum, insbesondere in einem kapillaren Raum, ermöglicht, wie er für die spätere Amplifikation der Nukleinsäuren benötigt wird. Ein hohes Verhältnis von Probevolumen zu Volumen des Bindungsraumes kann erhalten werden, wenn die Probe nicht, wie im Stand der Technik üblich, in den Bindungsraum eingefüllt wird, sondern vielmehr mindestens einmal, bevorzugt mehrmals durch den Bindungsraum hindurchgeleitet wird.

In einer weiteren bevorzugten Ausführungsform erfolgt die Bindung von Nukleinsäuren an die Oberfläche eines Glasvlieses in einer Packung, beispielsweise in einer Patrone, die an die oben beschriebene Kapillare angedockt wird. Auch bei dieser Ausführungsform wird die Nukleinsäuren enthaltende Probe bzw. die Lysemischung mindestens einmal durch die Packung geleitet, wobei die Nukleinsäuren beim Durchströmen an der Oberfläche des Glasvlieses binden.

Nach oder gleichzeitig mit dem Immobilisieren der Nukleinsäuren werden Verunreinigungen, die in der Probe enthalten sind, abgetrennt, wodurch ein selektiver und empfindlicher Nachweis von Nukleinsäuren erhalten wird. Die Abtrennung von Verunreinigungen kann beispielsweise dadurch erfolgen, daß der Bindungsraum, an den die Nukleinsäuren immobilisiert sind, mit einem Waschpuffer behandelt oder gespült wird. Der Waschpuffer wird bevorzugt langsam mindestens einmal durch den Bindungsraum geleitet und enthält bevorzugt 70 bis 80 Vol-% Ethanol.

Eine weitere Reinigung kann dadurch erfolgen, daß zur Entfernung des Waschpuffers der Bindungsraum getrocknet wird, indem er beispielsweise auf etwa 80°C aufgeheizt und gleichzeitig mit Luft oder einem Intergas durchgeströmt wird. Die Entfernung von Waschpufferresten, insbesondere von Restalkohol ist deswegen vorteilhaft, da Rückstände von Alkohol die anschließende Amplifikation inhibieren oder stören können.

Am Ende von Schritt (a) liegen die Nukleinsäuren bevorzugt in angetrockneter Form an den Bindungsraum immobilisiert, bevorzugt adsorptiv an eine Glasoberfläche gebunden vor.

Zur Vorbereitung der Amplifikation werden die immobilisierten Nukleinsäuren in Schritt (b) eluiert. Die Elution kann mit geeigneten, dem Fachmann bekannten Elutionslösungen durchgeführt werden. Nukleinsäuren können z.B. durch ein Reagenzgemisch mit niedrigem Salzgehalt von einer Glasoberfläche gelöst werden. Besonders vorteilhaft wird zum Eluieren eine Lösung eingesetzt, die bereits alle für die Amplifizierung erforderlichen Reagenzien enthält. In einer bevorzugten Ausführungsform wird durch Aufziehen eines Mastermix in den Bindungsraum, insbesondere eine Kapillare, die Nukleinsäure von der Oberfläche, insbesondere einer Glas- oder Polystyroloberfläche abgelöst. Unter Eluieren wird hier insbesondere das Ablösen der immobilisierten Nukleinsäuren von der Oberfläche verstanden, wobei die eluierten Nukleinsäuren nicht aus dem Bindungsraum entfernt werden. Werden in dem Probenvorbereitungsschritt die Infektionskeime beispielweise durch adsorbtive Bindung an Polystyrol aufgereinigt, werden diese bevorzugt erst zu Beginn des Amplifika-tionsschrittes lysiert. Es werden daher für den Probenvorbereitungsschritt nicht lysierende Puffer gebraucht.

Anschließend wird in Schritt (c) ein Amplifizieren der gereinigten Nukleinsäuren in einem Amplifikationsraum durchgeführt. Da der Amplifikationsraum mindestens einen Teil des Bindungsraumes umfaßt, ist eine Überführung oder ein Transport der eluierten Nukleinsäuren nicht erforderlich, so daß damit verbundene Ausbeuteverluste vermieden werden können. Die Amplifikation wird bevorzugt mittels Polymerase-Kettenreaktion (PCR) durchgeführt. Diese Amplifikationsmethode hat den Vorteil, daß sie universell einsetzbar ist, und eine hohe Spezifität und Sensitivität aufweist. Es können aber selbstverständlich auch andere, dem Fachmann bekannte Amplifikationsmethoden eingesetzt werden, wie beispielsweise IsoCR-, LCR-, Champ-, SDA-, Qβ-Replikase-, NASBA3SR-, CPT-, TMA-, rRNA-Hybridisierungs- oder bDNA-Methoden. Die jeweils am vorteilhaftesten einsetzbare Amplifikationsmethode kann in Abhängigkeit vom Analyten und anderen Rahmenbedingungen ohne weiteres vom Fachmann ermittelt werden.

Bevorzugt wird ein Amplifikationsraum verwendet, der thermostatisierbar ist. Es hat sich als besonders vorteilhaft herausgestellt, den Amplifikationsraum mit einer heizbaren Metallschicht zu umgeben, da auf diese Weise eine schnelle Erwärmung und somit kurze Heizzyklen möglich sind.

In einer bevorzugten Ausführungsform unter Verwendung der Polymerase-Kettenreaktion wird der Bindungsraum, insbesondere eine Kapillare an seinem unteren Ende zunächst mit einem ersten Stopfen verschlossen. Anschließend kann die Kapillare von der oben beschriebenen Doppelkolbenspritze abgenommen werden und mit einem zweiten Stopfen verschlossen werden. Der Bindungsraum dient dann gleichzeitigt als Amplifikationsraum.

Falls eine Kapillare mit einer heizbaren Metallschicht als Amplifikationsraum verwendet wird, wird beim Durchleiten von elektrischem Strom durch die Kapillare bzw. die elektrisch leitende Außenbeschichtung die Kapillare aufgeheizt. Die gewünschte Temperatur kann ohne weiteres anhand des Temperaturkoeffizienten und des elektrischen Widerstandes abgeleitet und geregelt werden. Die Abkühlung des Amplifikationsraumes kann beispielsweise durch Anblasen mit Umgebungsluft beschleunigt werden, wobei bei Verwendung einer Kapillare als Amplifikationsraum in Folge der geringen Masse nach Abschalten des Heizstromes ohnehin eine hohe Abkühlgeschwindigkeit erhalten wird. Bei der erfindungsgemäß bevorzugten Verwendung einer mit einer heizbaren Metallschicht umgebenen Kapillare ist aufgrund der hohen Aufheiz- und Abkühlgeschwindigkeiten ein Thermozyklus von 92°C, 55°C, 72°C in weniger als 25 sec durchführbar.

Die bei der Amplifikation gebildeten Produkte werden erfindungsgemäß detektiert, um die in der Probe enthaltenen Nukleinsäure nachzuweisen. Die Detektion kann nach bekannten Verfahren erfolgen. Bevorzugt erfolgt die Detektion online durch Fluoreszenz.

Besondere Vorteile hinsichtlich der Einfachheit des Verfahrens werden erhalten, wenn alle Schritte des Nachweisverfahrens, also das Reinigen der Nukleinsäuren, das Amplifizieren der gereinigten Nukleinsäuren und das Detektieren der Amplifikations-produkte im gleichen Reaktionsraum, insbesondere in einer Kapillare durchgeführt werden.

So kann bei der oben beschriebenen bevorzugten Ausführungsform einer mit Stopfen verschließbaren Kapillare als Bindungs- und Amplifikationsraum das Anregungslicht durch ein optisches Fenster in den Stopfen eingestrahlt und das durch ein Fenster am selben oder am gegenüberliegenden Stopfen auftretende Licht vermessen werden, um eine Fluoreszenzdetektion durchzuführen. In gleicher Weise kann auch die Änderung einer Adsorption zur Detektion herangezogen werden.

Zur Auswertung können dann die Intensitäten des Anregungslichtes und des Fluoreszenzlichtes miteinander verrechnet werden, wodurch ein Signal erhalten wird. Durch Auftrag der Signalintensität gegen die Anzahl der durchlaufenen Amplifikationszyklen kann neben dem qualitativen Nachweis auch quantitativ auf die Konzentration eines Analyten geschlossen werden. Dies ermöglicht eine diagnostische Vorentscheidung, beispielsweise aus dem Vergleich der Zyklenzahl, bei der ein deutlicher Signalanstieg beginnt, mit einem vorher definierten Grenzwert.

Eine weitere Verbesserung des Testes kann durch Zugabe einer definierten Menge eines internen Standards sowie der zugehörigen Sonden und Markierungen erzielt werden, wobei hier eine Überwachung der Amplifikation auf eine mögliche Inhibierung durch Testreagenzien oder/und in der Probe vorliegende Verunreinigungen durchgeführt werden kann. Vorteilhaft ist es hierbei, die interne Kontrolle und den Analyten auf zwei verschiedenen Wellenlängen nachzuweisen, was die gleichzeitige Durchführung von Analyse und Kontrolle ermöglicht.

Eine weitere Verfahrenskontrolle erhält man durch einen Vergleich der Temperaturkinetik beim Aufheizen des Amplifikationsraumes mit hinterlegten Sollwerten, woraus sich der Füllgrad des Amplifikationsraumes, insbesondere einer Kapillare ableiten läßt. Verliert der Amplifikationsraum während der Amplifikation Flüssigkeit, so führt das zu Änderungen der Aufheizkinetik und bei Überschreiten einer zuvor definierten Toleranzgrenze zur Fehlermeldung.

Zur weiteren Vereinfachung des Verfahrens werden bevorzugt alle Schritte in einer geschlossenen Vorrichtung ausgeführt, also in einer integrierten Vorrichtung, die alle notwendigen Reaktionsräume und Reagenzien enthält.

Das erfindungsgemäße Verfahren ist insbesondere für Point of Care (PoC)-Nachweise geeignet, da neben einer mit Labor-Standardmethoden vergleichbaren Leistungsfähigkeit eine einfache Handhabung ohne großen apparativen Aufwand, niedrige Herstellungskosten und ein geringer Analyseaufwand erzielt werden können. Durch die Integration der Probenvorbereitung mit der nachfolgenden Amplifikation können Pipettierschritte und damit verbundene Kontaminationsprobleme vermieden werden.

Das erfindungsgemäße Verfahren ist insbesondere zum Nachweis von Pathogenen in biologischen Proben geeignet. Es ermöglicht einen einfachen und schnellen Nachweis von Keimen, beispielsweise Chlamydia oder anderen.

Die Erfindung umfaßt weiterhin eine Vorrichtung zum Nachweis von Nukleinsäuren in einer Probe, insbesondere durch ein wie oben beschriebenes Verfahren, umfassend:
(a) einen Bindungsraum zur Reinigung von Nukleinsäuren, in dem die Nukleinsäuren immobilisiert und Verunreinigungen abgetrennt werden,
(b) einen Amplifikationsraum zur Amplifikation von Nukleinsäuren,
   wobei der Amplifikationsraum mindenstens einen Teil des Bindungsraumes umfasst
(c) einen Detektionsraum zur Detektion von Nukleinsäuren und gegebenenfalls
(d) Reservoirs oder/und Zuleitungen für Probe oder/und Reagenzien, welche dadurch gekennzeichnet ist, daß der Detektionsraum mindestens einen Teil des Amplifikationsraumes oder/und des Bindungsraums umfasst. Der Bindungs- und/oder Amplifikationsraum kann zumindest teilweise als kapillarer Raum ausgebildet sein, wodurch die oben beschriebenen Vorteile erhalten werden.

Eine Voraussetzung zum Nachweis von Nukleinsäuren in einer Probe besteht darin, eingeschlossene oder in Aggregaten gebundene Nukleinsäuren zunächst freizusetzen, um sie einer Analyse zugänglich zu machen. Hierzu werden beispielsweise Zellen mit lytischen Enzymen behandelt, um ein Aufbrechen der Zellwand und ein Freisetzen der in den Zellen enthaltenen Nukleinsäuren zu erreichen. Eine solche Lyse erfordert jedoch einen beträchtlichen Zeitaufwand.

WO 95/18851 beschreibt ein Verfahren zum Zerkleinern hochmolekularer Strukturen, wobei die Probe durch poröse Schichten geleitet wird, um die Zerkleinerung durch Scherung zu unterstützen. Hier besteht aber das Problem, daß auch der gewünschte Analyt zumindest teilweise in den Poren zurückgehalten wird und somit die Sensitivität des gesamten Nachweisverfahrens beeinträchtigt wird.

Zu illustrativen Zwecken wird ein Verfahren zum Aufschluß von nukleinsäurehaltigen Aggregaten beschrieben, welches eine Freisetzung der Nukleinsäuren in kurzer Zeit und ohne Ausbeuteverluste ermöglicht.

Dabei wird eine Nukleinsäuren enthaltende Matrix aufgeschlossen oder es werden nukleinsäurehaltige Aggregate aufgeschlossen, wobei eine Aufschlußmischung, die die Nukleinsäuren enthaltende Matrix und ein Aufschlußreagenz umfaßt, durch einen kapillaren Raum bewegt wird, wobei die Matrix aufgebrochen und die darin enthaltenen Nukleinsäuren freigesetzt werden.

Für einen Aufschluß von Nukleinsäuren enthaltenden Matrizen werden hohe Scherkräfte benötigt, um einen Probenaufschluß in kurzer Zeit zu erhalten. Beim erfindungsgemäßen Verfahren werden hohe Scherkräfte durch Verwendung eines kapillaren Raums und Bewegen der Probe, beispielsweise Durchleiten oder Fördern der Probe durch den kapillaren Raum erhalten, wodurch der Aufschluß der Nukleinsäure enthaltenden Matrix unterstützt wird.

Unter Aufschluß ist jedes Aufbrechen oder Zerkleinern einer Nukleinsäureenthaltenden Matrix zu verstehen, bei dem in der Matrix eingeschlossene oder mit der Matrix verbundene Nukleinsäuren freigesetzt werden. Beispielsweise handelt es sich bei dem Aufschluß um eine Lyse. Bei der Nukleinsäure enthaltenden Matrix handelt es sich z.B. um Zellen oder/und Zellfraktionen. Es können aber auch andere Gebilde aufgeschlossen werden, welche Nukleinsäuren eingeschlossen oder gebunden enthalten, wie etwa Micellen oder ähnliches.

Das Aufschlußreagenz dient dazu, die Freisetzung der Nukleinsäuren zu unterstützen. Bevorzugt wird ein Aufschlußreagenz verwendet das ein lytisches Enzym und/oder eine chaotrope Substanz enthält. Grundsätzlich sollte das Aufschlußreagenz in der Lage sein, die nukleinsäurehaltigen Aggregate aufzulösen oder anzugreifen.

Als kapillarer Raum wird z.B. eine Glaskapillare oder/und eine Polystyrol-Kapillare, insbesondere eine Borsilikat-beschichtete Kapillare verwendet. Dies hat den Vorteil, daß neben der Erzeugung von hohen Scherkräften, was den Aufschluß unterstützt, der gleiche Raum auch zur Immobilisierung und weiteren Behandlung bzw. Verarbeitung der freigesetzten Nukleinsäuren, wie oben beschrieben, verwendet werden kann. Bei dem kapillaren Raum kann es sich auch um einen Stempel mit kapillaren Löchern oder um einen Spalt zwischen einer Gefäßwand und einem Stempel handeln. Zum Beispiel wird die Probe mehrmals, insbesondere mehr als fünfmal, oder mehr als zehnmal durch den kapillaren Raum hindurchgeleitet, um einen schnellen Aufschluß zu erzielen. Das Volumenverhältnis von Aufschlußmischung zu kapillarem Raum kann dabei größer 10:1 oder größer als 20:1 sein. Wenn die Probe durch den kapillaren Raum hindurchgeleitet wird, ist es möglich, ein großes Probevolumen zu verwenden, was eine hohe Sensitivität ergibt und den kapillaren Raum klein zu halten, was Vorteile hinsichtlich der Gesamtanordnung und insbesondere hinsichtlich einer später durchzuführenden Amplifikation liefert.

Zu illustrativen Zwecken wird weiterhin ein Verfahren zur Gewinnung von Nukleinsäuren aus Mikroorganismen beschrieben, welches dadurch gekennzeichnet ist, daß man eine Mikroorganismen enthaltende Probe mit einer Polystyroloberfläche unter Bedingungen in Kontakt bringt, bei denen die Mikroorganismen an die Polystyroloberflächen binden und andere Probenbestandteile abtrennt und die Nukleinsäuren aus den Mikroorganismen gewinnt. Polystyroloberflächen sind hervorragend zur Probenvorbereitung für die Nukleinsäureanalytik geeignet. Gefäße, die eine Polystyroloberfläche aufweisen, können zur Probenvorbereitung bei der Nukleinsäureanalytik eingesetzt werden, wodurch eine hochintegrierte Anordnung bereitgestellt werden kann. Polystyrol bietet gegenüber den bisher zur Immobilisierung verwendeten Glasoberflächen zahlreiche Vorteile wie etwa leichte Verarbeitbarkeit, geringes Gewicht und mechanische Stabilität auch bei geringen Wandstärken.

Als Polystyroloberfläche ist beispielweise die Innenwand eines mit Polystyrol beschichteten oder aus Polystyrol bestehenden Bindungsraumes geeignet, aber auch die Verwendung von Polystyrolkügelchen oder Polystyrolbeads o.ä. Beispielsweise kann eine Polystyrol-Kapillare verwendet werden. Zur Erhöhung der Ausbeute und um den Bindungsraum möglichst klein zu halten, kann die Probe mehrmals über die Polystyroloberfläche geleitet werden.

Wie oben ausgeführt, ist es ein wesentliches Ziel bei der Entwicklung von Nukleinsäure-Nachweisverfahren, den apparativen und/oder zeitlichen Aufwand zu verringern. Die zur Durchführung einer Amplifikation benötigten Aufheiz- und Abkühlzyklen erfordern einen beträchtlichen Zeitaufwand, weshalb es vorteilhaft wäre, ein Verfahren bereitzustellen, bei dem dieser Zeitaufwand verringert ist.

Zu illustrativen Zwecken wird deshalb ein Verfahren zur Amplifikation von Nukleinsäuren beschrieben, das Schritte bei unterschiedlichen Temperaturen umfaßt, welches dadurch gekennzeichnet ist, daß die Amplifikation in einem Raum durchgeführt wird, der von einer heizbaren Metallschicht umgeben ist. Die Verwendung einer heizbaren Metallschicht, die den Amplifikationsraum bevorzugt vollflächig umgibt, können kurze Aufheizraten erzielt werden. Die Abkühlung kann dann auf herkömmliche Weise beschleunigt werden, beispielsweise durch das Anblasen mit Luft oder durch die Verwendung von anderen Kühltechniken. Die Amplifikation kann jedoch vorteilhaft in einem kapillaren Raum durchgeführt werden. Aufgrund der geringen Masse eines solchen kapillaren Raumes kann die Aufheiz- und Abkühlgeschwindigkeit weiter erhöht werden. Zum Beispiel kann eine Glas- oder/und Polystyrol-Kapillare verwendet werden, die von heizbaren, bevorzugt von einer vollflächigen heizbaren Metallschicht umgeben ist.

Weiterhin wird ein kapillares Reaktionsgefäß zur Durchführung einer Amplifikation von Nukleinsäuren betrachtet, welche von einer heizbaren Metallschicht umgeben ist.

Die oben genannten Aspekte der Erfindung können entweder allein oder in beliebiger Kombination verwendet werden, um eine Verbesserung der Nukleinsäureanalytik zu erhalten. Die genannten Verbesserungen sind insbesondere auch im Hinblick auf ein automatisierbares Gesamtverfahren von Vorteil. Eine einfache und schnelle Nukleinsäureanalyse kann insbesondere für Kontrollanalysen eingesetzt werden, die eine Aussage darüber ermöglichen sollen, ob die Durchführung umfangreicherer und aufwendigerer Testverfahren überhaupt indiziert ist. Die Verfahren sind auch für die gleichzeitige Untersuchung von mehreren Proben geeignet.

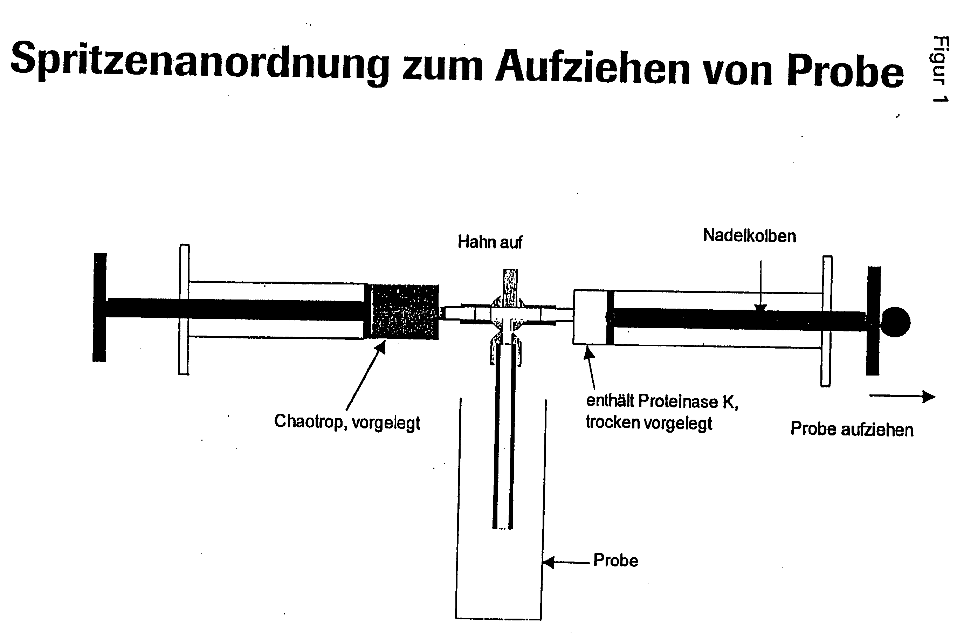

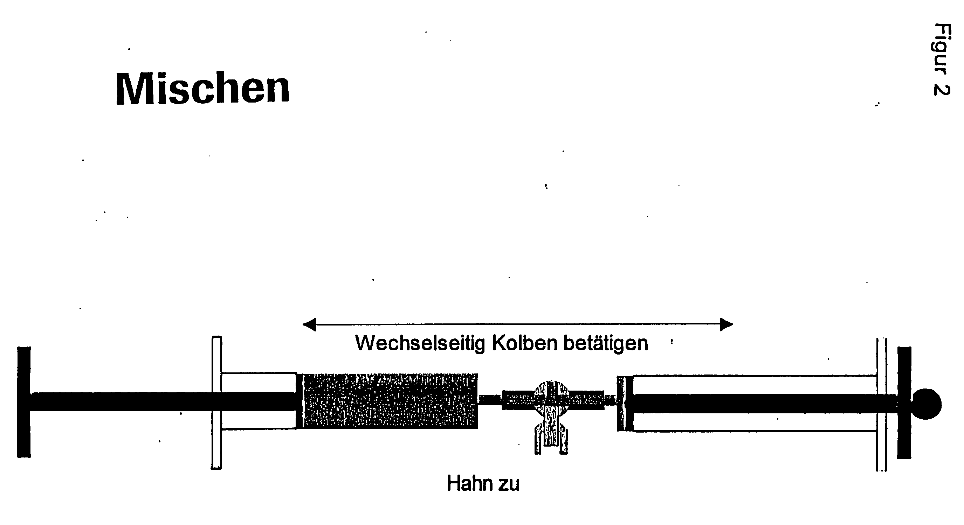

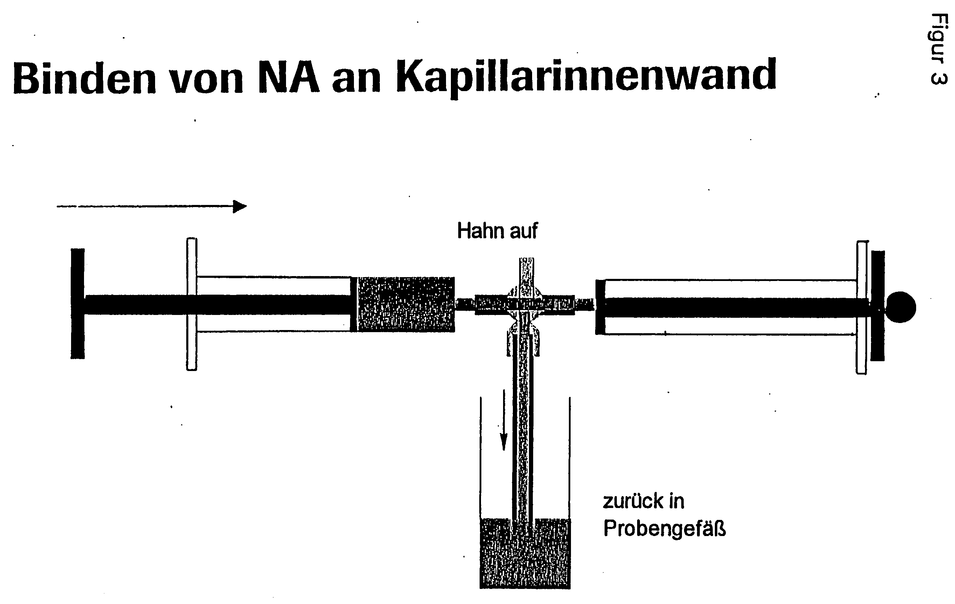

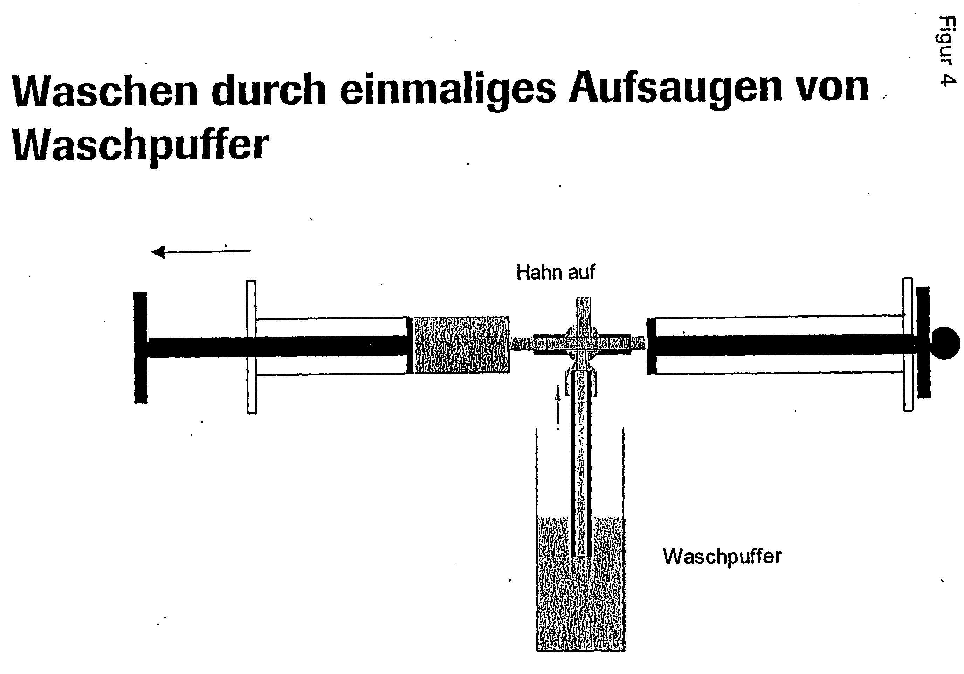

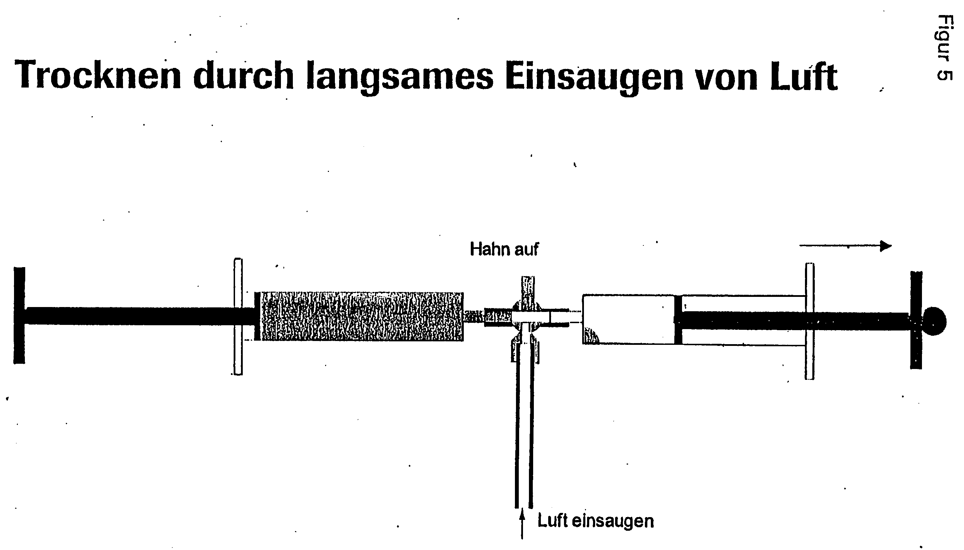

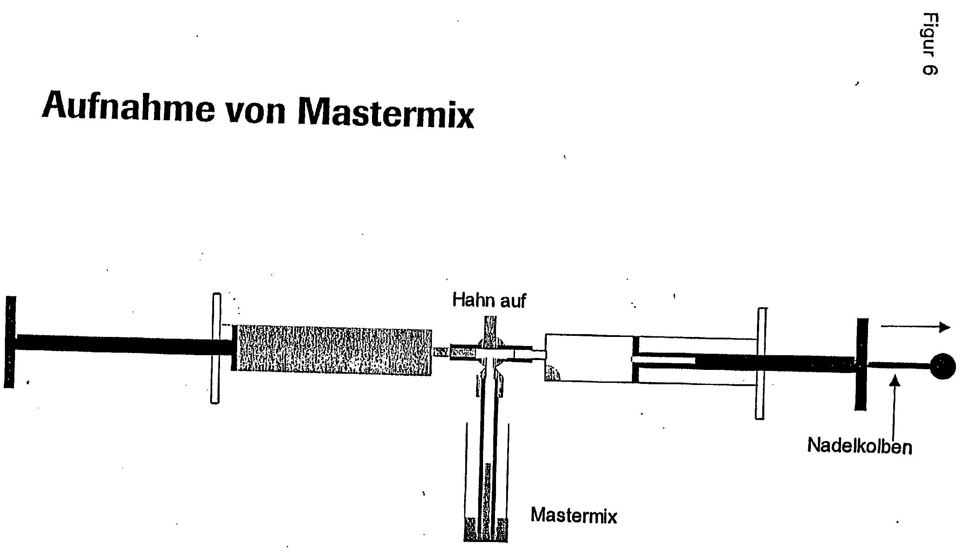

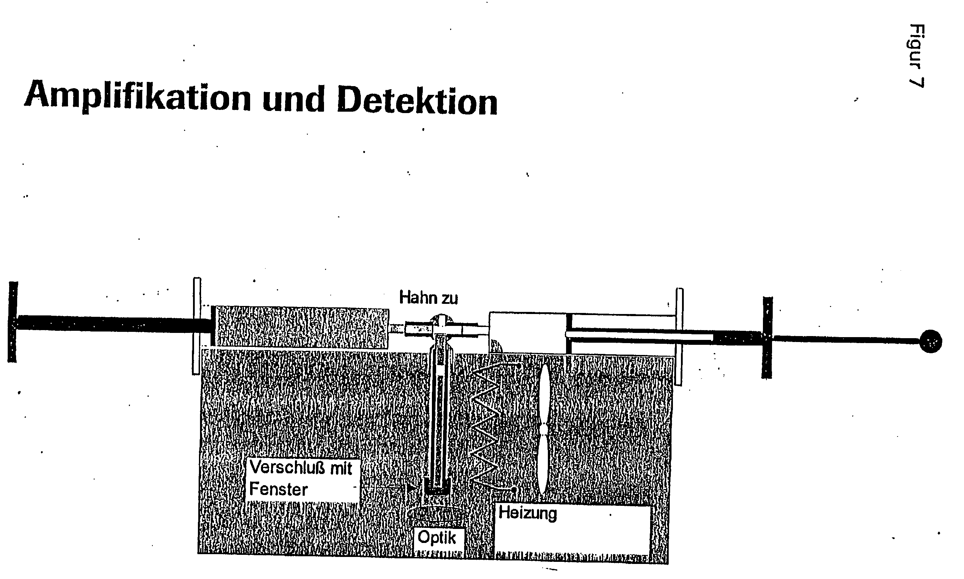

Die Erfindung wird durch die beigefügten Figuren und die nachfolgenden Beispiele weiter erläutert, worin die Figuren 1 bis 7 eine bevorzugte Durchführung des erfindungsgemäßen Verfahren zeigen.
- Fig. 1: zeigt eine Spritzenanordnung zum Aufziehen einer Zellen enthaltenden Probe, wobei in der linken Spritze ein Chaotrop vorgelegt ist, und in der rechten Doppelkammerspritze mit Nadelkolben Proteinase K trocken vorgelegt ist. Aus einem Vorratsgefäß wird die Probe durch Betätigung des Kolbens der rechten Spritze eingesaugt.
- Fig. 2: zeigt das Mischen der Probe mit den vorgelegten Reagenzien sowie den Aufschluß von nukleinsäurehaltigen Aggregaten. Dazu werden die Kolben der Spritzen wechselseitig betätigt und der Aufschluß durch Gleiten des Gemisches durch einen kapillaren Raum zwischen den Spritzen unterstützt.
- Fig. 3: zeigt das Binden der freigesetzten Nukleinsäure an eine Kapillarinnenwand. Dazu wird die Probe mindestens einmal, bevorzugt mehrmals, durch eine Kapillare zurück ins Probegefäß gedrückt und gegebenenfalls wieder eingesaugt.
- Fig. 4: zeigt das Waschen der an der Innenseite der Kapillare immobilisierten Nukleinsäuren durch einmaliges Aufsaugen eines Waschpuffers.
- Fig. 5: zeigt das Trocknen der gewaschenen immobilisierten Nukleinsäuren durch langsames Einsaugen von Luft.
- Fig. 6: zeigt die Aufnahme von Mastermix in die Kapillare, wobei der Mastermix die immobilisierten Nukleinsäuren eluiert und gleichzeitig alle für die nachfolgenden Amplifikation erforderlichen Reagenzien erhält.
- Fig. 7: zeigt die Amplifikation und Detektion der Amplifikationsprodukte. Dazu wird die Kapillare mit einem Verschlußstopfen verschlossen, welcher ein Fenster enthält. Nach Durchführung der Amplifikation, erfolgt die Detektion durch Einstrahlung von Licht über die gezeigte Optik (beispielsweise mittels Fluoreszenzdetektion).

### Beispiele

### 1. Doppelkolbenspritze zur Durchführung des erfindungsgemäßen Verfahrens

Die verwendete Doppelkolbenspritze entspricht weitgehend einer herkömmlichen Einmalspritze aus Polypropylen. Sie weist zwischen dem Spritzenkörper und dem Auslaß ein Ventil oder einen Absperrhahn auf. Der Doppelkolben ist so ausgeführt, daß im Inneren des Hauptkolbens die Schubstange für den Mischkolben läuft.

Der Hauptkolben weist an seinem unteren Ende zwei Dichtbereiche auf. Zum einen eine Dichtung zur Spritzenwand und zum anderen eine Dichtung zur Schubstange des Mischkolbens. Der Hauptkolben wird durch einen Schubzylinder bewegt, der über den Spritzenkörper hinausragt und an seinem oberen Ende so ausgeführt ist, daß er sicher mit der Hand oder alternativ mit einem Instrumententeil erfaßt und bewegt werden kann.

Der Mischkolben bildet an seinem unteren Ende mit der Spritzenwand einen schmalen Spalt. Die Schubstange des Mischkolbens läuft durch den Boden des Hauptkolbens und ragt bei jeder Stellung des Hauptkolbens noch aus dem Schubzylinder des Hauptkolbens. Am oberen Ende ist sie so ausgeführt, daß sie sicher mit der Hand oder alternativ einem Instrumententeil erfaßt und bewegt werden kann.

Der Absperrhahn kann wahlweise manuell oder automatisch betätigt werden, wobei er beide Stellungen (offen/geschlossen) dauerhaft einnehmen kann.

Die Öffnung der Spritze für Fluide ist in Form eines üblichen Konus ausgeführt. Günstigerweise ist der Konus passend zur Aufnahme von Einmalpipettenspitzen und der Amplifikationskapillare.

### 2. Durchführung des erfindungsgemäßen Verfahrens mit zwei separaten Spritzen

Anstelle der oben beschriebenen Doppelkolbenspritze können auch separate Spritzen, beispielsweise zwei Einmalspritzen aus Polypropylen über einen Kunststoffdreiwegehahn gekoppelt werden. Zum Aufziehen von Reagenz- bzw. Probe wird an den Dreiwegehahn über einen Konus eine Einmalpipettenspitze angedockt. Die Flüssigkeit wird dann durch Aufziehen der Spritzenkolben in eine der Spritzen aufgenommen. Es kann auch noch weitere Flüssigkeit in die gleiche Spritze gesaugt werden. Zum Mischen wird der Dreiwegehahn so gestellt, daß die beiden Spritzen verbunden sind und ein Austritt der aufgenommenen Probe bzw. Reagenzien verhindert wird. Durch wechselseitiges Betätigen der beiden Spritzenkolben wird die Flüssigkeit durch die Bohrung im Hahnkücken von einer zur anderen Spritze gedrückt und dabei unter hohen Scherkräften gemischt.

### 3. Volumenkontrolle des Amplifikationsgemisches

Um das Amplifikationsvolumen exakt in die Amplifikationskapillare aufzuziehen, wird einer der Kolben der Spritzen als Doppelkolben so ausgeführt, daß er zentral einen sehr schmalen Kolben aufnimmt, z.B. einen Kolben aus Metall- oder Kunststoffdraht, der einen Durchmesser entsprechend dem Innendurchmesser der Amplifikationskapillare aufweist. Damit ist eine genaue Aufnahme von Flüssigkeit in die Amplifikationskapillare möglich und es wird ein unbeabsichtigtes Einsaugen von Mastermix und Eluat in den Abfallbehälter vermieden.

### 4. Amplifikationskapillare

Die verwendete Amplifikationskapillare hat bevorzugt einen Innendurchmesser von ca. 1 mm und weist an der inneren Oberfläche eine Glasoberfläche auf. Die Glasoberfläche kann glatt oder zur Vergrößerung der Oberfläche aufgerauht oder strukturiert sein. Die Kapillare selbst kann aus Metall, Glas oder Kunststoff hergestellt sein, wobei die Wandung möglichst dünn ausgeführt ist. Die äußere Oberfläche ist aus elektrisch leitendem Material, bevorzugt aus Metall. Das elektrisch leitende Material hat einen elektrischen Widerstand mit einem positiven oder negativen Temperaturkoeffizienten. An beiden Enden der Kapillare befindet sich eine Beschichtung mit hoher elektrischer Leitfähigkeit und geringem Kontaktwiderstand zur Ausbildung eines elektrischen Kontakts zwischen der Kapillare und dem Instrument. Durch Anlegen einer elektrischen Spannung fließt Strom, der die leitfähige Schicht und damit die Kapillare erwärmt. Der Strom ist abhängig von der Temperatur und kann somit abhängig auch zur Temperaturmessung herangezogen werden.

Die Amplifikationskapillare wird bevorzugt mit Verschlußstoffen versehen, welche aus Kunststoff, insbesonder einem optisch klaren Material gebildet sind. Besonders bevorzugt sind Polycarbonat und Polypropylen. Günstigerweise ist der Verschlußstopfen so ausgeführt, daß ein Stopfen in den den Innenraum der Kapillare ragt und das Äußere von einem Hemd umfaßt wird. Durch die der Kapillare abgewandte Seite kann Licht ausgestrahlt bzw. eingeleitet werden. Dazu kann in den Verschlußstopfen ein optisches Element, beispielsweise ein Fenster oder eine Linse integriert sein. Die die Wandung des Hemdes des Stopfens kann zusätzlich ein elektrisch leitendes Element, z.B. ein Draht integriert sein, der beim Aufdrücken des Stopfens auf die Kapillare einen elektrischen Kontakt herstellt.

### 5. Spritze mit Glasvlies als Bindephase (Spritzenvorsatzfilter)

Zur Bindung von Nukleinsäuren nach chaotroper Lyse an eine Glasvlies-Oberfläche wurde ein Glasvlies in einen Spritzenvorsatzfilter integriert. Der direkte Einsatz des Glasvlieses in den Amplifikationsraum ist aufgrund einer möglichen Inhibition des Glasvliesmaterial während der Amplifikationsreaktion, z.B. einer PCR-Reaktion nicht vorteilhaft. 250 µl Chlamydien-haltiger Zellkultur-Überstand (1%) wurden durch chaotrope Lyse lysiert. Das Lysat wurde mittels einer Einmalspritze über das Glasvlies geleitet, um ein Binden der Nukleinsäuren zu bewirken. Als Lyse-, Wasch- und Elutionspuffer wurden die entsprechenden Lösungen des High Pure Plasmid Isolation Kits (Boehringer Mannheim Kat. No. 1754777) verwendet. Das bei den Versuchen eingesetzte Glasvlies war in seiner Zusammensetzung identisch mit dem Glasvlies der Filtertubes des High Pure Plasmid Isolation Kits. Anschließend wurde mit Waschpuffer gewaschen und mit Luft getrocknet. Zu beachten ist hierbei, daß Alkoholreste aus dem Waschpuffer möglichst vollständig entfernt werden. Anschließend wurden die Nukleinsäuren eluiert und eine PCR-Amplifikation und eine Gel-Detektion durchgeführt. Bevorzugt wird die Elution der Nukleinsäure mit der Master-Lösung durchgeführt, die bereits alle für die Amplifikation erforderlichen Reagenzien enthält. Es wurde zwar festgestellt, daß die Vliespassage zu einem Amplifikationsverlust bei der PCR-Reaktion führt (10⁵-CT-Plasmide); dies wird allerdings durch die hohe Bindekapazität und hohe Bindegeschwindigkeit des Glasvliesmaterials kompensiert.

### PCR-Protokoll

Als Primer für die Amplifikation wurden die Oligonukleotide CP24 5'-GGGATTCCTGTAACAACAAGTCAGG-3' (Position 195-219 von pCTT1) und CP27: 5'-CCTCTTCCCCAGAACAATAAGAACAC-3' (Position 401-376 von pCTT1) gegebenenfalls im 5'-biotinylierter oder 5'-digoxigenylierter Form verwendet.

Das Reaktionsvolumen war 100 µl (4 mM MgCl2, jeweils 0,1 mM dNTP, jeweils 300 nM Primer CP24 und CP27, 2,5 U Taq-Polymerase, 2 U UNG (Uracil-DNA-Glycosylase) und Matrize in PCR-Puffer (Roche Diagnostics Katalog-Nr. 1600753).

Die Reaktionsführung war wie folgt:
- 10 min 37°C, 5 min bei 95°C, 1 min bei 60°C
- 34 Zyklen mit jeweils 30 s bei 95°C und 60 s bei 60°C
- 10 min bei 72°C
- Halten bei 50°C

### 6. Spritze mit Glaskapillare als Bindephase

Ein wesentlicher Vorteil bei der Probenvorbereitung in einer Glaskapillare, beispielsweise in Kombination mit einer Spritze, besteht in der einfachen manuellen Bedienbarkeit sowie der einfachen Integration mit der nachfolgenden Amplifikation. Die bei der Probenvorbereitung verwendete Kapillare kann mit den daran gebundenen Nukleinsäuren nach Befüllen mit Mastermix direkt für die Amplifikation verwendet werden. Der Probenvorbereitungsraum ist somit zugleich Amplifikationsraum. Die Verwendung einer Glaskapillare ermöglicht sowohl eine schnelle Amplifikation als auch eine Online-Detektion und stellt somit eine hochintegrierte Gesamtlösung für den Nukleinsäuren-Nachweis dar.

Das Probematerial wird bevorzugt mehrmals durch die Glaskapillare geleitet, beispielsweise mit einer Schlauchpumpe bei Inkubationszeiten von etwa 20 min. Es ist aber auch möglich, die Probe-enthaltende Lösung mittels einer manuell bedienenden Spritze mit einer Inkubationszeit von lediglich 1 min durch die Kapillare zu leiten, wobei immer noch eine ausreichende Sensitivität des Tests erzielt wird. Besonders vorteilhaft ist es, auch hier zur Eluierung der immobilisierten Nukleinsäuren direkt eine Mastermix-Lösung zu verwenden, so daß der Amplifikationsschritt, beispielsweise eine PCR unter Wegfall eines separaten Elutionsschrittes durchgeführt werden kann.

### 7. Spritze mit Polystyrolkapillare als Bindephase

Es wurde festgestellt, daß Mikroorganismen in wässrigem Milieu an Polystyroloberflächen gebunden werden können. So binden beispielweise Chlamydien an eine Polystyrol-Einmalimpföse in einem 20minütigen Inkubationsschritt und können direkt in einem PCR-Reaktionsansatz überführt werden. Es wurden deshalb Polystyrolkapillaren in Verbindung mit einer Einmalspritze eingesetzt, um eine hochintegrierte Vorrichtung für einen Chlamydien-Nukleinsäurenachweis herzustellen. Bei einer Inkubationszeit einer Chlamydien-enthaltenden Probe von 1 min konnte je nach Verdünnungsmedium (H₂O, PBS oder Urin) eine Sensitivität zwischen 0,1 bis 0,01 % Zellkultur-Überstand erhalten werden.

Zur Herstellung von Polystyrolkapillaren wurden Standard-Polystyrol-Reaktionsgefäße (Sarstedt) nach Erwärmen zu Kapillaren ausgezogen und in Kombination mit einer Einmalspritze verwendet. Es zeigte sich, daß die Polystyrolkapillaren hervorragend zur Probenvorbereitung von Nukleinsäuren geeignet sind. Bei einer solchen Probenvorbereitung gelang es 300 µl eines 0,01 % Zellkulturüberstands in einer nachfolgenden PCR-Reaktion und Detektion noch deutlich als positiv nachzuweisen. Es gelang somit, unter Verwendung einer Polystyrolkapillare eine herkömmlichen Labormethoden vergleichbare Sensitivität zu erzielen.

### 8. Probenvorbereitung mit Glaskapillare

Als Glaskapillare wurde eine 5 µl Modulohm-Kapillare (Borsilikatglas) mit 3 cm Länge verwendet. 250 µl Probe + 50 µl Proteinase K (20 mg/ml) und 250 µl Lysereagenz (5,4 M GuSCN, 20% Triton X-100, 1% DTT, 10 mM Tris HCI, pH 6) wurden nach kurzem vortexen für 10 min bei 70°C inkubiert und anschließend über 5 min auf Raumtemperatur abkühlen gelassen. Eine Spritze (10 ml, Becton Dickinson) wurde über einen kurzen Kunststoffschlauch mit der Glaskapillare verbunden. Das Lysat wurde über 2 min mit gleichmäßiger Bewegung in die Spritze aufgezogen und ausgestoßen. Während dieser Zeit fand eine Bindung der Nukleinsäuren an die Kapillare statt. Dann wurden 800 µl Waschpuffer (20 mM NaCI, 10 mM Tris HCI pH 7,5, 70 Vol-% Ethanol) mit der Spritze über 2 min durch die Kapillare gespült und die Kapillare anschließend durch Aufziehen von Luft über 1 min getrocknet. Dann wurden 100 µl Elutionspuffer (10 mM Tris HCI pH 8,5) mit der zweiten Spritze (1 ml; Fa. Becton Dickinson) in die Kapillare aufgezogen. Die PCR-Reaktion erfolgte bei den in 5 angegebenen Bedingungen. Nachgewiesen wurde mittels einer Nachweissonde (5'-GTCTCTCATCGAGACAAAGTG-3'ausdemChlamydiatrachomatis-Plasmid pCTT [C.thrachomatis Basen 1-7496] entsprechend Position 354-374 von pCTT1 (Sriprakash und Macavoy, Plasmid 18 (1987), 205-214) nach Standardvorgehensweise.

### 9. Probenvorbereitung mit einer Polystyrolkapillare

Polystyrolgefäße (Sarstedt) wurden mit einer Heizlampe erhitzt und zu Kapillaren mit einem Durchmesser zwischen 1 und 2 mm ausgezogen. Nach dem Erkalten wurden 3 cm lange Teilstücke abgetrennt. Eine Spritze (10 ml, Bectonc Dickinson) wurde über einen kurzen Kunststoffschlauch mit der Polystyrolkapillare verbunden. Dann wurde die Probe für 1 min mittels der Spritze durch die Kapillare gespült. Anschließend wurden 800 µl Waschpuffer mittels der Spritze durch die Kapillare gezogen und die Kapillare durch Aufziehen von Luft für 1 min getrocknet. Die Amplifikation mittels einer PCR-Reaktion und Detektion mittels Hybridisierung mit einer Nachweissonde wie unter 8 beschrieben können anschließend direkt unter Verwendung der Polystyrolkapillare oder durch Zerschneiden der Kapillare und Überführen in ein PCR-Reaktionsgefäß durchgeführt werden.

## Patentansprüche

1. Verfahren zum Nachweis von Nukleinsäuren in einer Probe umfassend die Schritte:
(a) Reinigen der Nukleinsäuren in einem Bindungsraum, wobei die Nukleinsäuren immobilisiert und Verunreinigungen abgetrennt werden,
(b) Eluieren der immobilisierten Nukleinsäuren,
(c) Amplifizieren der gereinigten Nukleinsäuren in einem Amplifikationsraum, wobei der Amplifikationsraum mindestens einen Teil des Bindungsraumes umfasst,
(d) Detektieren der Amplifikationsprodukte in einem Detektionsraum,
**dadurch gekennzeichnet,**
**dass** der Detektionsraum mindestens einen Teil des Amplifikationsraumes oder/und mindestens einen Teil des Bindungsraumes umfasst.

2. Verfahren nach Anspruch 1,
**dadurch gekennzeichnet,**
**daß** als Bindungs- oder/und Amplifikationsraum ein zumindest teilweise kapillarer Raum verwendet wird.

3. Verfahren nach einem der vorhergehenden Ansprüche,
**dadurch gekennzeichnet,**
**daß** in Schritt (a) eine Adsorption von Nukleinsäuren an eine Glasoberfläche durchgeführt wird.

4. Verfahren nach einem der vorhergehenden Ansprüche,
**dadurch gekennzeichnet,**
**daß** zum Eluieren in Schritt (b) eine Lösung eingesetzt wird, die alle für die Amplifizierung erforderlichen Reagenzien enthält.

5. Verfahren nach einem der vorhergehenden Ansprüche,
**dadurch gekennzeichnet,**
**daß** der Amplifikationsraum thermostatisierbar ist.

6. Verfahren nach Anspruch 5,
**dadurch gekennzeichnet,**
**daß** der Amplifikationsraum mit einer heizbaren Metallschicht umgeben ist.

7. Verfahren nach Anspruch 6,
**dadurch gekennzeichnet,**
**dass** der Amplifikationsraum von einer vollflächigen Metallschicht umgeben ist.

8. Verfahren nach einem der Ansprüche 6 oder 7,
**dadurch gekennzeichnet,**
**dass** als Amplifikationsraum eine Glas- oder/und Polystyrol-Kapillare verwendet wird, die von einer heizbaren Metallschicht umgeben ist.

9. Verfahren nach einem der vorhergehenden Ansprüche,
**dadurch gekennzeichnet,**
**daß** vor dem Reinigen der Nukleinsäuren in Schritt (a) ein Aufschluss oder eine Lyse von nukleinsäurehaltigen Proben durchgeführt wird.

10. Verfahren nach Anspruch 9,
**dadurch gekennzeichnet,**
**daß** zum Aufschluß einer Nukleinsäure enthaltenden Matrix eine Aufschlußmischung umfassend die Nukleinsäure enthaltende Matrix und ein Aufschlußreagenz, durch einen kapillaren Raum bewegt wird, wobei die Matrix aufgebrochen und die darin enthaltenen Nukleinsäuren freigesetzt werden.

11. Verfahren nach Anspruch 9 oder 10,
**dadurch gekennzeichnet,**
**daß** ein Aufschlußreagenz verwendet wird, das ein lytisches Enzym oder/und eine chaotrope Substanz enthält.

12. Verfahren nach einem der Ansprüche 9 bis 11,
**dadurch gekennzeichnet,**
**daß** es sich bei dem kapillaren Raum um eine Glaskapillare oder/und eine Polystyrol-Kapillare handelt.

13. Verfahren nach Anspruch 12,
**dadurch gekennzeichnet,**
**daß** es sich bei dem kapillaren Raum um eine Borsilikat-beschichtete Kapillare handelt.

14. Verfahren nach einem der Ansprüche 9 bis 13,
**dadurch gekennzeichnet,**
**daß** die Probe mehrmals durch den kapillaren Raum hindurch geleitet wird.

15. Verfahren nach einem der Ansprüche 9 bis 14,
**dadurch gekennzeichnet,**
**daß** das Volumenverhältnis von Aufschlußmischung zu kapillarem Raum größer als 10 : 1 ist.

16. Verfahren nach einem der vorhergehenden Ansprüche,
**dadurch gekennzeichnet,**
**dass** die Probe Zellen oder/und Zellfraktionen umfasst.

17. Verfahren nach Anspruch 16,
**dadurch gekennzeichnet,**
**dass** die Zellen an eine Polystyroloberfläche gebunden werden.

18. Verfahren nach Anspruch 17,
**dadurch gekennzeichnet,**
**daß** man zur Gewinnung von Nukleinsäuren aus Mikroorganismen eine Mikroorganismen enthaltende Probe mit einer Polystyroloberfläche unter Bedingungen in Kontakt bringt, bei denen die Mikroorganismen an die Polystyroloberfläche binden, andere Probenbestandteile abtrennt, und die Nukleinsäuren aus den Mikroorganismen gewinnt.

19. Verfahren nach Anspruch 18,
**dadurch gekennzeichnet,**
**daß** weiterhin ein Salz zugesetzt wird, um die Bindung der Mikroorganismen an die Polystyroloberfläche zu unterstützen.

20. Verfahren nach Anspruch 18 oder 19,
**dadurch gekennzeichnet,**
**daß** man eine Polystyrol-Kapillare verwendet.

21. Verfahren nach einem der Ansprüche 18 bis 20,
**dadurch gekennzeichnet,**
**daß** die Probe mehrmals über die Polystyrol-Oberfläche geleitet wird.

22. Verfahren nach einem der Ansprüche 18 bis 21,
**dadurch gekennzeichnet,**
**daß** es sich bei den Mikroorganismen um Chlamydien handelt.

23. Verfahren nach einem der Ansprüche 18 bis 22,
**dadurch gekennzeichnet,**
**daß** Urin als Probe verwendet wird.

24. Verfahren nach einem der vorhergehenden Ansprüche,
**dadurch gekennzeichnet,**
**dass** das Reinigen der Nukleinsäuren, das Amplifizieren der gereinigten Nukleinsäuren und das Detektieren der Amplifikationsprodukte im gleichen Reaktionsraum durchgeführt werden.

25. Verfahren nach einem der vorhergehenden Ansprüche,
**dadurch gekennzeichnet,**
**dass** alle Schritte in einer geschlossenen Vorrichtung durchgeführt werden.

26. Verwendung des Verfahrens nach einem der Ansprüche 1 bis 25 zum Nachweis von Pathogenen in biologischen Proben.

27. Vorrichtung zum Nachweis von Nukleinsäuren in einer Probe, insbesondere durch ein Verfahren nach einem der Ansprüche 1 bis 25, umfassend:
(a) einen Bindungsraum zur Reinigung von Nukleinsäuren, in dem die Nukleinsäuren immobilisiert und Verunreinigungen abgetrennt werden,
(b) einen Amplifikationsraum zur Amplifikation von Nukleinsäuren, wobei der Amplifikationsraum mindestens einen Teil des Bindungsraumes umfasst,
(c) einen Detektionsraum zur Detektion von Nukleinsäuren und gegebenenfalls
(d) Reservoirs oder/und Zuleitungen für Probe oder/und Reagenzien, **dadurch gekennzeichnet, dass** der Detektionsraum mindestes einen Teil des Amplifikationsraums oder/und des Bindungsraums umfasst.

28. Vorrichtung nach Anspruch 26 oder 27,
**dadurch gekennzeichnet,**
**daß** der Bindungs- oder/und Amplifikationsraum zumindest teilweise als kapillarer Raum ausgebildet ist.

29. Vorrichtung nach Anspruch 28,
**dadurch gekennzeichnet,**
**dass** der Amplifikationsraum von einer heizbaren Metallschicht umgeben ist.

## Claims

1. A method for detecting nucleic acids in a sample comprising the steps:
(a) purifying the nucleic acids in a binding chamber, wherein the nucleic acids are immobilised and impurities are separated,
(b) eluting the immobilised nucleic acids,
(c) amplifying the purified nucleic acids in an amplification chamber, wherein the amplification chamber comprises at least a part of the binding chamber,
(d) detecting the amplification products in a detection chamber,
**characterised in that** the detection chamber comprises at least a part of the amplification chamber and/or at least a part of the binding chamber.

2. A method according to Claim 1,
**characterised in that** at least part of a capillary chamber is used as the binding chamber and/or amplification chamber.

3. A method according to one of the preceding Claims,
**characterised in that** in step (a) an adsorption of nucleic acids is carried out on to a glass surface.

4. A method according to any one of the preceding Claims,
**characterised in that** a solution, which contains all the reagents required for the amplification, is used for the elution in step (b).

5. A method according to any one of the preceding Claims,
**characterised in that** the amplification chamber can be thermostatically controlled.

6. A method according to Claim 5,
**characterised in that** the amplification chamber is surrounded by a heatable metal layer.

7. A method according to Claim 6,
**characterised in that** the amplification chamber is surrounded by a complete metal layer.

8. A method according to either Claim 6 or Claim 7,
**characterised in that** a glass and/or polystyrene capillary is used as the amplification chamber, which is surrounded by a heatable metal layer.

9. A method according to any one of the preceding Claims,
**characterised in that** a break-down or lysis of samples containing nucleic acids is carried out before the purification of the nucleic acids in step (a).

10. A method according to Claim 9,
**characterised in that**, for the break-down of a nucleic acid-containing matrix, a break-down mixture comprising the nucleic acid-containing matrix and a break-down reagent is passed through a capillary chamber, wherein the matrix is broken down and the nucleic acids contained therein are released.

11. A method according to Claim 9 or 10, **characterised in that** a break-down reagent is used which contains a lytic enzyme and/or a chaotropic substance.

12. A method according to any one of Claims 9 to 11,
**characterised in that** the capillary chamber is a glass capillary and/or a polystyrene capillary.

13. A method according to Claim 12,
**characterised in that** the capillary chamber is a capillary coated with boron silicate.

14. A method according to any one of the Claims 9 to 13,
**characterised in that** the sample is passed several times through the capillary chamber.

15. A method according to any one of the Claims 9 to 14,
**characterised in that** the volume ratio of break-down mixture to capillary chamber is higher than 10:1.

16. A method according to any one of the preceding Claims,
**characterised in that** the sample comprises cells and/or cell fractions.

17. A method according to Claim 16,
**characterised in that** the cells are bound to a polystyrene surface.

18. A method according to Claim 17,
**characterised in that**, to obtain nucleic acids from microorganisms, a sample containing microorganisms is contacted with a polystyrene surface under conditions in which the microorganisms bind to the polystyrene surface and other sample components are separated, and the nucleic acids are obtained from the microorganisms.

19. A method according to Claim 18,
**characterised in that** a salt is additionally added so as to facilitate the binding of the microorganisms to the polystyrene surface.

20. A method according to Claim 18 or 19, **characterised in that** a polystyrene capillary is used.

21. A method according to any one of Claims 18 to 20,
**characterised in that** the sample is passed several times over the polystyrene surface.

22. A method according to any one of Claims 18 to 21,
**characterised in that** the microorganisms are Chlamydia.

23. A method according to any one of Claims 18 to 22,
**characterised in that** urine is used as the sample.

24. A method according to any one of the preceding Claims,
**characterised in that** the purification of the nucleic acids, the amplification of the purified nucleic acids and the detection of the amplification products is carried out in the same reaction chamber.

25. A method according to any one of the preceding Claims,
**characterised in that** all the steps are carried out in a closed apparatus.

26. A method according to any one of the Claims 1 to 25 for detecting pathogens in biological samples.

27. An apparatus for detecting nucleic acids in a sample, in particular by a method according to any one of the Claims 1 to 25, comprising:
(a) a binding chamber for purifying nucleic acids, in which the nucleic acids are immobilised and impurities are separated,
(b) an amplification chamber for amplifying nucleic acids, wherein the amplification chamber comprises at least a part of the binding chamber,
(c) a detection chamber for detecting nucleic acids and, optionally,
(d) reservoirs and/or supply lines for the sample and/or reagents,
**characterised in that** the detection chamber comprises at least a part of the amplification chamber and/or of the binding chamber.

28. An apparatus according to Claims 26 or 27,
**characterised in that** the binding chamber and/or amplification chamber is at least partly in the form of a capillary chamber.

29. An apparatus according to Claims 28,
**characterised in that** the amplification chamber is surrounded by a heatable metal layer.

## Revendications

1. Procédé de détection d'acides nucléiques dans un échantillon, comprenant les étapes consistant à :
(a) purifier les acides nucléiques dans un espace de liaison où les acides nucléiques sont immobilisés et les impuretés sont séparées,
(b) élution des acides nucléiques immobilisés,
(c) amplification des acides nucléiques purifiés dans un espace d'amplification, l'espace d'amplification comprenant au moins une partie de l'espace de liaison,
(d) détection du produit d'amplification dans un espace de détection,
**caractérisé en ce que** l'espace de détection comprend au moins une partie de l'espace d'amplification et/ou au moins une partie de l'espace de liaison.

2. Procédé selon la revendication 1, **caractérisé en ce qu'**on utilise comme espace de liaison et/ou d'amplification, un espace au moins partiellement capillaire.

3. Procédé selon l'une quelconque des revendications précédentes, **caractérisé en ce que** dans l'étape (a), on réalise l'adsorption des acides nucléiques sur une surface en verre.

4. Procédé selon l'une quelconque des revendications précédentes, **caractérisé en ce que**, pour l'élution de l'étape (b), on met en oeuvre une solution qui contient tous les réactifs nécessaires à l'amplification.

5. Procédé selon l'une quelconque des revendications précédentes, **caractérisé en ce que** l'espace d'amplification peut être thermostaté.

6. Procédé selon la revendication 5, **caractérisé en ce que** l'espace d'amplification est entouré d'une couche métallique.

7. Procédé selon la revendication 6, **caractérisé en ce que** l'espace d'amplification est entouré d'une couche métallique sur toute sa surface.

8. Procédé selon l'une des revendications 6 ou 7, **caractérisé en ce que** comme espace d'amplification, un capillaire en verre et/ou en polystyrène est utilisé, qui est enveloppé d'une couche métallique pouvant être chauffée.

9. Procédé selon l'une quelconque des revendications précédentes, **caractérisé en ce qu'**avant la purification des acides nucléiques à l'étape (a), on réalise une dégradation ou une lyse des échantillons contenant les acides nucléiques.

10. Procédé selon la revendication 9, **caractérisé en ce que**, pour la dégradation d'une matrice contenant des acides nucléiques, on fait passer un mélange de dégradation comprenant la matrice contenant les acides nucléiques et un réactif de dégradation dans un espace capillaire de manière à rompre la matrice et libérer les acides nucléiques contenus dans celle-ci.

11. Procédé selon la revendication 9 ou 10, **caractérisé en ce que** l'on utilise un réactif de dégradation qui contient une enzyme lytique et/ou une substance chaotrope.

12. Procédé selon l'une des revendications 9 à 11, **caractérisé en ce que** l'espace capillaire est un capillaire en verre et/ou un capillaire en polystyrène.

13. Procédé selon la revendication 12, **caractérisé en ce que** l'espace capillaire est un capillaire enduit de borosilicate.

14. Procédé selon l'une des revendications 9 à 13, **caractérisé en ce que** l'on fait passer l'échantillon plusieurs fois dans l'espace capillaire.

15. Procédé selon l'une des revendications 9 à 14, **caractérisé en ce que** le rapport volumique du mélange de dégradation à l'espace capillaire est supérieur à 10:1.

16. Procédé selon l'une quelconque des revendications précédentes,
**caractérisé en ce que** l'échantillon comprend des cellules et/ou des fragments de cellule.

17. Procédé selon la revendication 16, **caractérisé en ce que** les cellules sont fixées sur une surface en polystyrène.

18. Procédé selon la revendication 17, **caractérisé en ce que**, pour l'obtention des acides nucléiques à partir de microorganismes, on met en contact un échantillon contenant les microorganismes avec une surface en polystyrène, dans des conditions dans lesquelles les microorganismes se fixent à la surface en polystyrène, les autres constituants sont séparés et on obtient les acides nucléiques des microorganismes.

19. Procédé selon la revendication 18, **caractérisé en ce que** l'on ajoute en outre, un sel pour favoriser la fixation des microorganismes sur la surface en polystyrène.

20. Procédé selon la revendication 18 ou 19, **caractérisé en ce que** l'on utilise un capillaire en polystyrène.

21. Procédé selon l'une des revendications 18 à 20, **caractérisé en ce que** l'on fait passer l'échantillon plusieurs fois sur la surface en polystyrène.

22. Procédé selon l'une des revendications 18 à 21, **caractérisé en ce que** les microorganismes sont des Chlamydies.

23. Procédé selon l'une des revendications 18 à 22, **caractérisé en ce que** l'on utilise de l'urine comme échantillon.

24. Procédé selon l'une quelconque des revendications précédentes,
**caractérisé en ce que** la purification des acides nucléiques, l'amplification des acides nucléiques purifiés, et la détection du produit d'amplification sont réalisées dans le même espace de réaction.

25. Procédé selon l'une quelconque des revendications précédentes,
**caractérisé en ce que** toutes les étapes sont réalisées dans un dispositif fermé.

26. Utilisation du procédé selon l'une des revendications 1 à 25, pour la détection de pathogènes dans des échantillons biologiques.

27. Dispositif de détection d'acides nucléiques dans un échantillon, en particulier par un procédé selon l'une des revendications 1 à 25, comprenant :
(a) un espace de liaison pour la purification d'acides nucléiques, dans lequel les acides nucléiques sont immobilisés et les impuretés sont séparées,
(b) un espace d'amplification, pour l'amplification des acides nucléiques, l'espace d'amplification comprenant au moins une partie de l'espace de liaison,
(c) un espace de détection pour la détection des acides nucléiques, et le cas échéant
(d) des réservoirs et/ou conduites pour les échantillons et/ou réactifs,
**caractérisé en ce que** l'espace de détection comprend au moins une partie de l'espace d'amplification et/ou de l'espace de liaison.

28. Dispositif selon la revendication 26 ou 27, **caractérisé en ce que** l'espace de liaison et/ou d'amplification sont formés au moins partiellement, comme un espace capillaire.

29. Dispositif selon la revendication 28, **caractérisé en ce que** l'espace d'amplification est enveloppé d'une couche métallique pouvant être chauffée.
